# NEW EUROPEAN PATENT SPECIFICATION

(11) **EP 2 736 482 B2**
(45) Date of publication and mention of the opposition decision: **13.09.2023**
(45) Mention of the grant of the patent: 14.12.2016
(21) Application number: 12759212.9
(22) Date of filing: 19.07.2012
(51) Int. Cl.: A61Q 17/04, A61K 8/34, A61K 8/37, A61K 8/41, A61K 8/49, A61K 8/67, A61K 8/92

(54) **ECO-COMPATIBLE SUNSCREEN COMPOSITIONS**
ECO-KOMPATIBLE SONNNENSCHUTZZUSAMMENSETZUNGEN
COMPOSITIONS D'ÉCRAN SOLAIRE ECO-COMPATIBLES

(30) Priority: 27.07.2011 IT RM20110400
(43) Date of publication of application: 04.06.2014
(73) Proprietor: Aethic Ltd., London W9 3QJ (GB)
(72) Inventor: DANOVARO, Roberto, I-60121 Ancona (IT); DAMIANI, Elisabetta, I-60131 Ancona (IT); CORINALDESI, Cinzia, I-60131 Ancona (IT)
(74) Representative: Di Giovine, Paolo
(86) International application number: PCT/IB2012/053694
(87) International publication number: WO 2013/014584

(56) References cited:
- EP-A1- 1 591 104
- EP-A1- 2 087 521
- EP-A2- 1 179 339
- WO-A1-2007/083174
- DE-A1- 19 726 783
- DE-A1-102008 018 788
- FR-A1- 2 943 541
- US-A1- 2007 202 060
- DATABASE GNPD [Online] Mintel; 1 October 2010 (2010-10-01), "Sunscreen", XP002671828, Database accession no. 1414626
- DATABASE GNPD [Online] Mintel; 1 May 2011 (2011-05-01), "Anti age intensive cream", XP002671829, Database accession no. 1553846
- DANOVARO ROBERTO ET AL: "Sunscreens cause coral bleaching by promoting viral infections.", ENVIRONMENTAL HEALTH PERSPECTIVES APR 2008 LNKD- PUBMED:18414624, vol. 116, no. 4, April 2008 (2008-04), pages 441-447, XP002671830, ISSN: 0091-6765
- DATABASE GNPD [Online] MINTEL; 17 June 2011 (2011-06-17), ANONYMOUS: "Replenishing solar defense spf 30", XP002711369, retrieved from www.gnpd.com Database accession no. 1619121
- "Orangenol - vielseitig einsetzbar vom Raumduft bis zur Hautpflege", Orangenol, pages 1-2, Retrieved from the Internet: URL:www.orangenoel.info [retrieved on 2017-08-23]
- R. Danovaro et al,'Sunscreens cause coral bleaching by promoting viral infections', Environmental Health Perspectives, 116(4), 2008, 441-7

## Description

The present description refers to eco-compatible sunscreen compositions, in particular to sunscreens compatible with the marine environment and the life inhabiting it, with specific reference to coral reefs.

### STATE OF THE PRIOR ART

Coral reefs are among the most biologically productive and diverse ecosystems in the world, representing hot spots of marine biodiversity, and directly sustaining half a billion people (Moberg and Folke 1999, Wilkinson 2004). Approximately 60% of coral reefs are currently threatened by several natural and anthropogenic impacts (Hughes et al 2003; Pandolfi et al 2003). Over the last 20 years, coral bleaching (loss of symbiotic zooxanthellae hosted within scleractinian corals) has increased dramatically, both in frequency and spatial extent (Hoegh-Guldberg 1999; Hughes et al 2003; Knowlton 2001). This phenomenon has been associated with temperature anomalies, excess ultraviolet (UV) radiation or altered availability of photosynthetic radiation, and presence of bacterial pathogens and pollutants (Brown et al 2000; Bruno et al 2007; Douglas 2003; Glynn 1996; Jones 2004).

Production and consumption of personal care and cosmetic sun products are increasing worldwide, with potentially important consequences related to environmental contamination. The release of these products is also linked with the rapid expansion of tourism in marine coastal areas (Wilkinson 2004). Chemical compounds contained in sunscreens and other personal care products can reach detectable levels in seawater (Daughton and Ternes 1999; Giokas et al 2007). These compounds might be potentially harmful to the environment; hence the use of sunscreen products is now banned in a few of the most popular tourist destinations, for example, in marine ecoparks in Mexico (Xcaret 2007; Xel-ha 2007). Because sunscreens are lipophilic, their UV filters can accumulate in aquatic animals (Giokas et al, 2007) and cause effects similar to those reported for other xenobiotic compounds (Balmer et al 2005; Daughton and Ternes 1999).

Paraben preservatives and some UV absorbers contained in sunscreens have estrogenic activity (Daughton and Ternes 1999; Schlumpf et al 2004). In addition, it has been demonstrated that sunscreen ingredients can undergo various photodegradations, with the entailed transformation of these agents into toxic byproducts (Giokas et al. 2007, and literature therein). It has also been demonstrated that sunscreens have an impact on marine bacteria (Danovaro and Corinaldesi 2003). Recently, a tight relationship among sunscreens and bleaching of hard corals was observed through specific tests worldwide (Danovaro et al. 2008). In particular, in this study the harmful effects on corals of some UV filters used in sunscreens and of some commercially available sunscreen compositions have been analyzed.

A product manufactured by the company MyChelle is a non-toxic reef-safe sunscreen composition comprising the Zinc Oxide as a sunblock.

Object of the present invention is that of solving the above-mentioned drawbacks by providing a sunscreen composition that is eco-compatible with the marine ecosystem of the coral reef.

### SUMMARY OF THE INVENTION

Commercially available sunscreens comprise a variety of chemical compounds, synthetic and natural ones, all potentially harmful to the marine environment of the coral reef. The Inventors have surprisingly selected some substances that, both alone and in combination among them, cause no harm in this type of marine environment, but concomitantly are highly effective in screening the skin from solar radiations.

Therefore, object of the present invention is an eco-compatible sunscreen composition according to claims 1-11.

### BRIEF DESCRIPTION OF THE FIGURES

**Figure 1****.** Viral enrichment factors in water surrounding coral nubbins ("small branches with alive polyps") in treated systems and in control systems (controls). The various classes of different treatments tested are shown: a) Fragrances; b) Creams; c) Antioxidants; d) UV filters; e) Controls.
**Figure 2****.** Prokaryotic enrichment factors in water surrounding coral nubbins in treated and control systems. The various classes of different treatments tested are shown: a) Fragrances; b) Creams; c) Antioxidants; d) UV filters; e) Controls.
**Figure 3****.** Total zooxanthellae released in water surrounding nubbins during the incubation test. The various classes of different treatments tested are shown: a) Fragrances; b) Creams; c) Antioxidants; d) UV filters; e) Controls.
**Figure 4****.** Level of damage of zooxanthellae released after addition of sunscreen Creams; c) Antioxidants; d) UV filters; e) Controls.

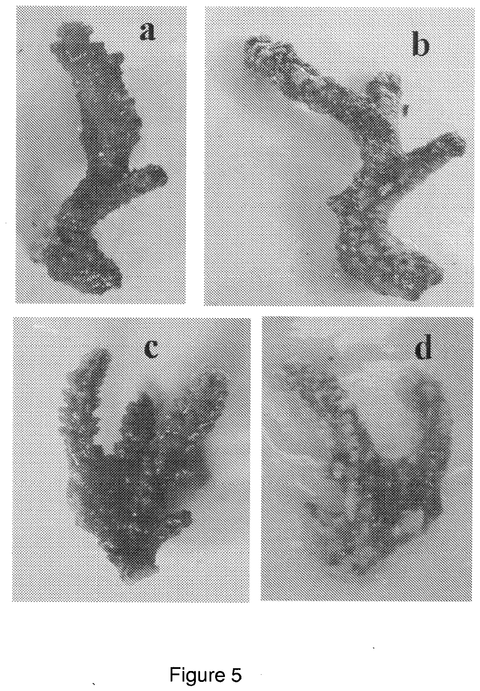

**Figure 5****.** Coral nubbins at different incubation times. Panels a and b show control systems at time 0 and after 72 h of incubation. Panels c and d show nubbins of treated corals at time 0 and after 72 h of incubation.

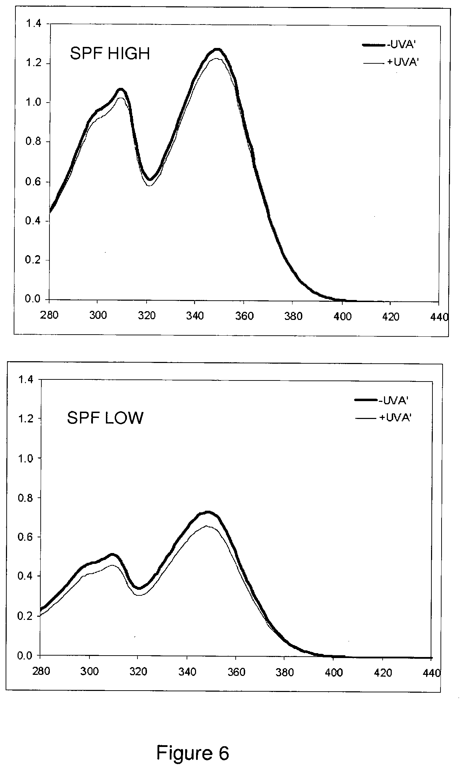

**Figure 6****.** Photostability of UV-tested filters.

### DETAILED DESCRIPTION OF THE INVENTION

The present invention refers to sunscreen compositions eco-compatible with the marine ecosystem of coral reefs as defined in the claims.

In the present description, by the definition: "eco-compatible with the marine ecosystem" of the coral reefs it is meant a product causing no harm nor alteration to the marine organisms or to the communities or habitats as a whole. An eco-compatible product is therefore usable without restrictions, as it protects the health of environment and biodiversity. In the tests reported herein, the health conditions of marine organisms exposed to the various chemical components were verified in order to select the components absolutely innocuous. Note that a biodegradable product (declaration associated with some marketed products) can be harmful to the environment and therefore can be not eco-compatible, as the concept of biodegradation refers only to the decomposition times of the compound.

The compositions of the present invention are defined in claims 1, 2, 3 and 11.

In the present description, by "parabens" are meant the esters of 4-hydroxybenzoic acid used as preservatives in the cosmetics industry. The most common parabens are methylparaben, ethylparaben, propylparaben and butylparaben.

An example of polyphenols extracted from green tea is polyphenone-60 (CAS N° 138988-88-2) commercially available from Sigma.

In an embodiment, the compositions of the present invention may comprise one or more fragrances eco-compatible with the marine environment, selected from the group consisting of fragrances of orange, lavender, grapefruit, guava and coconut.

Examples of fragrances suitable for the present invention are the fragrances commercially available from FAROTTI SRL company, such as guava and coconut, natural lavender, natural grapefruit, preferably in concentrations equal to or lower than 0.3% of the product.

In order to increase the shelf life times, in an embodiment the compositions comprise as preservative sorbic acid, which from the testing reported herein did not prove harmful to coral reefs in conjunction with the other compounds.

The compositions could be prepared in different cosmetic forms, such as, e.g., cream, lotion, ointment, spray, lipstick. The selected UV filters, antioxidants, fragrances and preservatives could be mixed with different carriers suitable (according to test results) for the determined cosmetic form that is to be manufactured.

Preferably, carriers having an emollient function will be used, selected in the group consisting of propylene glycol dicaprylate/dicaprate, olive oil, beeswax.

Propylene glycol dicaprylate/dicaprate (CAS number: 68583-51-7) is a derivative of propylene glycol, and is a diester of propylene glycol and fatty acids, In particular, it is a mixture of propylene glycol dicaprylate and propylene glycol dicaprate. This product is commercially available under the name Labrafac.

Beeswax (CAS number:8012-89-3) suitable for the present invention is e.g. commercially available under the name Apifil.

The data reported in the experimental section demonstrate that the filters MBBT, DHHB, the antioxidants tocopheryl acetate and tocopherol and the fragrance of guava and coconut are the compounds having a greater eco-compatibility with corals. Preferably, the compositions of the present invention will therefore comprise MBBT or DHHB, tocopheryl acetate or Tocopherol and optionally the fragrance of guava and coconut.

The present invention is based on the selection of compounds suitable to be used in compositions for use as sunscreens compatible with the marine environment and life inhabiting it, with particular reference to coral reefs.

The majority of compounds commonly used in sunscreens proved harmful to these environments, compounds such as, e.g.:
- preservatives belonging to the paraben family
- ascorbic acid derivatives;
- UV filters selected from bis ethylhexyloxyphenol methoxyphenyl triazine (BEMT), cinnamates, benzophenones, camphor derivatives, titanium oxide;
- fragrances selected from melaleuca essential oil, ylang ylang flower extracts, Bambousa Arundinacea extracts, Karitè;
- carriers selected from animal-derived fats;
- Argan oil.

In an embodiment, the sunscreen compositions comprise:
- UV filters: 2,2'-methylene-bis-(6-(2H-benzotriazole-2-yl)-4-(1,1,3,3-tetramethylbutyl)-phenol, 2-[4-(diethylamine)-2-hydroxybenzoyl]hexyl benzoate and ethylhexyl triazone;
- one or more antioxidants selected from tocopheryl acetate, tocopherol, retinol, polyphenolic compounds extracted from green tea;
- one or more carriers selected from propylene glycol dicaprylate/dicaprate, olive oil, beeswax
and are free from:
- preservatives belonging to the paraben family or ascorbic acid derivatives;
- UV filters selected from bis ethylhexyloxyphenol methoxyphenyl triazine, cinnamates, benzophenones, camphor derivatives, titanium oxide;
- fragrances selected from melaleuca essential oil, ylang ylang flower extracts, Bambousa Arundinacea extracts, Karitè;
carriers selected from animal-derived fats and argan oil.

In an embodiment with a very high eco-compatibility, they will further comprise sorbic acid and/or one or more fragrances selected from orange, lavender, grapefruit, guava and coconut.

In a further highly eco-compatible embodiment the compositions are essentially consisting of:
- one or more UV filters selected from 2,2'-methylene-bis-(6-(2H-benzotriazole-2-yl)-4-(1,1,3,3-tetramethylbutyl)-phenol, 2-[4-(diethylamine)-2-hydroxybenzoyl]hexyl benzoate, ethylhexyl triazone;
- one or more antioxidants selected from tocopheryl acetate, tocopherol, retinol, polyphenolic compounds extracted from green tea;
- one or more carriers selected from propylene glycol dicaprylate/dicaprate, olive oil, beeswax;
- water.

In a further highly eco-compatible embodiment, the compositions are essentially consisting of:
- one or more UV filters selected from 2,2'-methylene-bis-(6-(2H-benzotriazole-2-yl)-4-(1,1,3,3-tetramethylbutyl)-phenol, 2-[4-(diethylamine)-2-hydroxybenzoyl]hexyl benzoate, ethylhexyl triazone;
- one or more antioxidants selected from tocopheryl acetate, tocopherol, retinol, polyphenolic compounds extracted from green tea;
- sorbic acid;
- one or more carriers selected from propylene glycol dicaprylate/dicaprate, olive oil, beeswax;
- water.

In a further embodiment, the compositions are essentially consisting of:
- 2,2'-methylene-bis-(6-(2H-benzotriazole-2-yl)-4-(1,1,3,3-tetramethylbutyl)-phenol and/or 2-[4-(diethylamine)-2-hydroxybenzoyl]hexyl benzoate;
- tocopheryl acetate and/or tocopherol;
- sorbic acid;
- one or more carriers selected from propylene glycol dicaprylate/dicaprate, olive oil, beeswax;
- water;
- one or more fragrances selected from orange, lavender, grapefruit, guava and coconut.

In a further embodiment, the compositions are essentially consisting of:
- 2,2'-methylene-bis-(6-(2H-benzotriazole-2-yl)-4-(1,1,3,3-tetramethylbutyl)-phenol and/or 2-[4-(diethylamine)-2-hydroxybenzoyl]hexyl benzoate;
- tocopheryl acetate and/or tocopherol;
- sorbic acid;
- one or more carriers selected from propylene glycol dicaprylate/dicaprate, olive oil, beeswax;
- water;
- one or more fragrances selected from orange, lavender, grapefruit, guava and coconut;
- hydroxyethyl cellulose and/or EDTA and/or sodium hydroxide.

In a further embodiment, the compositions are essentially consisting of:
- 2,2'-methylene-bis-(6-(2H-benzotriazole-2-yl)-4-(1,1,3,3-tetramethylbutyl)-phenol, 2-[4-(diethylamine)-2-hydroxybenzoyl]hexyl benzoate and ethylhexyl triazone;
- tocopheryl acetate;
- sorbic acid;
- propylene glycol dicaprylate/dicaprate, olive oil and beeswax;
- water;
- hydroxyethyl cellulose;
- EDTA;
- sodium hydroxide.

All embodiments listed above are exclusively consisting of compounds tested individually and as a whole to have a full eco-compatibility with the coral reef and (with no need of addition of other compounds potentially harmful for the marine ecosystem) have a high photostability and prolonged storage in a suitable cosmetic formulation.

In the compositions of the present invention, the MBBT, DHHB and EHT could be in a percentage comprised between 3 and 6% by weight of the composition.

### EXAMPLES

The following examples are merely illustrative and do not limit the invention (e.g., the weighings and concentrations are merely indicative, as the SPFs and the UVA/UVB absorbance ratios have to be specifically formulated to conform to the nominal SPFs of the product). Amounts of individual compounds are expressed as weight percentages.

### Example 1: In a specific embodiment, the invention has the following formulation in the form of cream:

| **Ingredients** | **w/w** |
|---|---|
| MBBT | 4% |
| DHHB | 4% |
| EHT | 1% |
| APIFIL | 12% |
| LABRAFAC | 15.5% |
| Olive oil | 7% |
| Tocopheryl acetate | 0.5% |
| Hydroxyethyl cellulose | 0.2% |
| EDTA | 0.05% |
| Sodium hydroxide | 0.4% |
| Sorbic acid | 1% |
| Guava and coconut | 0.3% |
| H₂O | remaining % |

### Example 2: In a specific embodiment, the invention has the following formulation in the form of cream:

| **Ingredients** | **w/w** |
|---|---|
| MBBT | 6% |
| DHHB | 6% |
| EHT | 2% |
| APIFIL | 11% |
| LABRAFAC | 14.5% |
| Olive oil | 6% |
| Tocopheryl acetate | 0.5% |
| Hydroxyethyl cellulose | 0.2% |
| EDTA | 0.05% |
| Sodium hydroxide | 0.4% |
| Sorbic acid | 1% |
| Lavender | 0.3% |
| H₂O | remaining % |

### Example 3: In a specific embodiment, the invention has the following formulation in the form of cream:

| **Ingredients** | **w/w** |
|---|---|
| MBBT | 8% |
| DHHB | 8% |
| EHT | 3% |
| APIFIL | 10% |
| LABRAFAC | 13.5% |
| Olive oil | 5% |
| Tocopheryl acetate | 0.5% |
| Hydroxyethyl cellulose | 0.2% |
| EDTA | 0.05% |
| Sodium hydroxide | 0.4% |
| Sorbic acid | 1% |
| Orange | 0.3% |
| H₂O | remaining % |

### Example 4: preparing the compositions

Compositions are prepared by the process described hereinafter:
Phases 1 and 3, indicated in Table 1 are dissolved together at 65°C using a stirrer.
Phase 2 is dissolved separately at the same temperature, always under continuous stirring. Upon reaching the same temperature, phase 2 is added to phases 1+3 and the whole is mixed for some minutes at 65°C. To obtain a product having homogeneous consistency, it is mixed at room temperature and only at the end, then phase 4 is added. The three compositions thus prepared were tested as described in the experimental section of the present description.

**Table 1. Composition of the 3 preparations of examples 1, 2 and 3 having a different SPF.**

| | **Base** | **1 - low SPF** | **2 - medium SPF** | **3 - high SPF** |
|---|---|---|---|---|
| **Phase 1** | Apifil 13% | Apifil 12% | Apifil 11% | Apifil 10% |
| | Labrafac 18.5% | Labrafac 15.5% | Labrafac 14.5% | Labrafac 13.5% |
| | Olive oil 8% | Olive oil 7% | Olive oil 6% | Olive oil 5% |
| | Tocopheryl acetate 0.5% | Tocopheryl acetate 0.5% | Tocopheryl acetate 0.5% | Tocopheryl acetate 0.5% |
| | | DHHB4% | DHHB 6% | DHHB8% |
| | | EHT 1% | EHT 2% | EHT 3% |
| | | MBBT4% | MBBT 6% | MBBT 8% |
| **Phase 2** | Water 57.22 | Water 53.05 % | Water 51.05% | Water 49.05% |
| | Hydroxyethyl cellulose 0.2% | Hydroxyethyl cellulose 0.2% | Hydroxyethyl cellulose 0.2% | Hydroxyethyl cellulose 0.2% |
| | EDTA 0.05% | EDTA 0.05% | EDTA 0.05% | EDTA 0.05% |
| | Sorbic acid 1% | Sorbic acid 1% | Sorbic acid 1% | Sorbic acid 1% |
| **Phase 3** | Labrafac 1 % | Labrafac 1 % | Labrafac 1 % | Labrafac 1 % |
| | Sodium hydroxide 0.4% | Sodium hydroxide 0.4% | Sodium hydroxide 0.4% | Sodium hydroxide 0.4% |
| **Phase 4** | Fragrance 0.3% | Fragrance 0.3% | Fragrance 0.3% | Fragrance 0.3% |

### METHODOLOGY AND EXPERIMENTAL DATA

### 3.1. Study area

Field activities were conducted at the Marine Biology Laboratory of Onong Resort, Siladen Island, Indonesia. The study area is located in Bunaken National Park, considered one of the most important Marine Protected Areas in the world. Bunaken National Park is located in the centre of a reef-building coral biodiversity triangle, which roughly encompasses the Philippines, Indonesia and the Great Barrier Reef. In total, about 605 zooxanthellate coral species were recorded in the Coral Triangle (Veron et al., 2009), of which 66% are common to all ecoregions of the area. This diversity amounts to 76% of the world's total species. About 450 coral species have been described so far in the Indonesian archipelago. Bunaken National Park and adjacent waters support diverse hermatypic (reef-building) coral communities, composed of more than 390 species, 63 genera and 15 families of hard corals, including also some species yet undescribed. The corals form different community types, broadly distributed in relation to depth, slope angle and other environmental factors. The area supports various regionally important populations of a wide array of coral species, including some 20 species considered to be globally rare. Bunaken National Park exhibits exceptional levels of within-location diversity. Richest locations host more than 25 - 30 % of the reef-building coral species complement of the entire Indo-Pacific region. In particular, in the study area, recent surveys reported that the reef-building corals accounted for about 90% of total coral species, with highest coral diversity occurring in shallow waters, likely related to the relatively higher habitat heterogeneity produced by changes in slope angle, exposure and illumination among other factors, from the shallow reef slope to the crest to the reef flat. In the study area, the occurrence of many endemic species of the genus Acropora were recently reported (Wallace et al., 2001). Acropora represent one of the larger taxonomic group in the reefs surrounding Siladen Island. Acropora is the most diverse hermatypic coral genus, with 114 species recognised worldwide and 91 species being identified for the Indonesian archipelago (Wallace, 1999). "Structural species" such as Acropora are a vital ecosystem component, and variations in their abundance are critical to the dynamics of entire reef communities (Connell et al., 1997). Acropora spp. plays a dominant role in the species composition and abundance of many modern day Indonesian reefs (Suharsono, 1992).

### 3.2. Experimental design

*In situ* tests were performed on nubbins (i.e., small branches of about 3-6 cm) of the stony coral *Acropora sp.* The nubbins were incubated in microcosms in presence of known concentrations of various ingredients potentially to be included in the composition of the eco-compatible sunscreen cream. In particular, coral nubbins (including more than 3000 polyps each) were collected by scuba divers from the same colony, located at a depth of about 3 m on the edge of the reef slope. Sampling of nubbins was performed by means of forceps. The nubbins were washed with seawater filtered onto 0.02-µm filters to eliminate viruses and prokaryotes. Each microcosm was prepared with a replicate set of nubbins (n=3) immersed in sterile polyethylene bags (Whirl-pack, Nasco, Fort Atkinson, WI, USA) filled with 2 L filtered seawater. A total of 27 microcosms were prepared, in order to test 20 ingredients with the respective positive and negative controls. The list of treatments, the concentrations of compounds used and the control systems are reported in Table 2. The microcosms were incubated at *in situ* temperature and light conditions for 72 hours.

**Table 2. List of microcosms, treatment types, concentrations of compounds used and incubation times.**

| **Code** | **Compound** | **Concentration** | **Type** |
|---|---|---|---|
| D-01 | Natural grapefruit fragrance | 0.3 % | **Essences/ Fragrances** |
| D-02 | Tea Tree oil *(Melaleuca)* fragrance | 0.3 % | |
| D-03 | Ylang-ylang fragrance I | 0.3 % | |
| D-04 | Natural orange fragrance | 0.3 % | |
| D-05 | Natural lavender fragrance | 0.3 % | |
| D-06 | Argan oil | 0.3 % | |
| D-07 | Guava & Coconut 22661 | 0.3 % | |
| D-08 | Bamboo 21842 | 0.3 % | |
| D-09 | Karite 20471 | 0.3 % | |
| D-10 | *Mare d'inverno* 4/381 MC | 0.3 % | |
| D-11 | *Joyful* 60029MC | 0.3 % | |
| D-13 | Type-1 base cream | 50 µL⁻¹ | **Base cream** |
| D-14 | Type-2 base cream | 50 µL⁻¹ | |
| D-15 | Type-3 base cream | 50 µL⁻¹ | |
| D-16 | Alpha-tocopherol (SIGMA code T3251-5G) | 3% | **Antioxidant** |
| D-17 | DL-tocopherol-acetate (SIGMA code T3376-5G) | 3% | |
| D-18 | 2-phospho-ascorbic acid trisodium salt (SIGMA code 49752-10G) | 3% | |
| D-19 | retinyl palmitate (SIGMA code R3375-1G) | 3% | |
| D-20 | Retinol (SIGMA code R7632-250MG) | 3% | |
| D-21 | Polyphenol 60 from green tea (SIGMA code P1204-25G) | 3% | |
| D-22 | MBBT (filter) | 7% | **Filters** |
| D-23 | BEMT (filter) | 7% | |
| D-24 | EHT (filter) | 3% | |
| D-25 | DHHB (filter) | 7% | |
| Mito | Mitomycin C (agent inducing the lytic cycle in viral infections). | 1 µg mL⁻¹ | **Control systems** |
| BLK | - | | |
| BLK PG | PG | | |

Both negative and positive control systems (controls) were used. The negative ones are systems in which a substance has been used, the antibiotic mitomycin C, which triggers viral infections, as it happens with use of all creams currently marketed. Therefore, it serves to indicate the levels of undesired response of a compound. The positive ones, denoted by BLK PG and BLK, are simply microcosms without addition of any compound.

**Table 3. Concentrations of tested filters**

| **FILTERS** | **Max concentration** | **Low SPF** | **Medium SPF** | **High SPF** |
|---|---|---|---|---|
| MBBT | 10% | 4 | 6 | 8 |
| DHHB | 10% | 4 | 6 | 8 |
| EHT | 5% | 1 | 2 | 3 |

### 3.3. Determination of viral and prokaryotic abundances

Subsamples (15 mL) of seawater surrounding coral nubbins were collected at the start of the experiment (TO) and after 3 and 6 hours of incubation. After subsampling, sea water was transferred into sterile test tubes without use of fixatives and stored at -20°C until laboratory analysis for quantification of viral and prokaryotic abundance. Once in laboratory, the water samples were immediately processed. Briefly, 200 µL sea water were diluted 1:10 with prefiltered MilliQ water, through 0.02-µm pore filters (Anodisc filter; 25-mm diameter, Al₂O₃; Whatman) and stained with 20 µL SYBR Green (stock solution diluted 1:20; Invitrogen, Carlsbad, CA, USA). Filters were incubated in the dark for 15 min and mounted on glass slides with a drop of antifade consisting of 50% phosphate buffer (6.7 mM, pH 7.8) and 50% glycerol containing 0.25% ascorbic acid (Shibata et al. 2006; Helton et al. 2006; Wen et al. 2004). Slides were stored at -20°C until microscopy analysis. Virus and prokaryote counts were obtained by epifluorescence microscopy (magnification, ×1000; Zeiss Axioplan) by examining at least 10 optical fields, that is, at least 200 cells or viruses per replicate. Viral (and prokaryotic) enrichment was calculated as ratio between virus (and prokaryote) abundances and virus (and prokaryote) abundance at the end of the experiment.

### 3.4 Zooxanthellae counts

Subsamples (50 mL) of seawater surrounding coral nubbins were collected at the start of the experiment (TO) and after 6, 12, 24, 36, 48 and 72 hours of incubation. The subsamples were fixed in 3% glutaraldehyde and then about 6.0 ml of each replicate was filtered through 2.0-µm polycarbonate filters. Filters were mounted on glass slides and zooxanthellae counted under epifluorescence microscopy (Zeiss Axioplan, Carl Zeiss Inc., Jena, Germany; × 400 and ×1000) using standard and UV light. Based on color, autofluorescence and gross cell structure, zooxanthellae released or extracted from nubbins were classified as a) healthy (H, brown/bright yellow color, intact/in good conditions zooxanthellae; b) pale (P, pale yellow color, vacuolated, partially degraded zooxanthellae; transparent (T, lacking pigmentations, empty zooxanthellae; Mise and Hidaka 2003).

### 3.5 Quantification of coral bleaching

In order to quantify the levels of coral bleaching (Siebeck et al. 2006) a colorimetric analysis was performed on digital photographs of corals, taken at the beginning of the experiment and after 6, 12, 24, 36, 48 and 72 hours of incubation. Photographs were taken under identical illumination with a Canon PowerShot A620 digital camera (Canon Inc., Tokyo, Japan) with a scale meter on the background. The photos were successively analyzed with a photo-editing software (Adobe Photoshop CS2) for color composition [cyan, magenta, yellow, black (CMYK)]. Levels of bleaching were measured as the difference between the corals' color at the beginning of the experiment and after treatments. Variations in the percentage of the different color components (CMYK) were analyzed with one-way analysis of variance (ANOVA). To rank the bleaching effect due to the compounds tested, a Bray-Curtis similarity matrix was prepared and a multidimensional scaling analysis of the shifts in CMYK color composition of treated corals was performed using Primer 5.0 software (Primer-E Ltd., Plymouth, UK). Bleaching rates were measured as the dissimilarity percentage in CMYK color composition between treated and control corals using the SIMPER tool of Primer 5.0 software (Primer-E Ltd).

### 4. Results

### 4.1. Viral and prokaryotic abundance

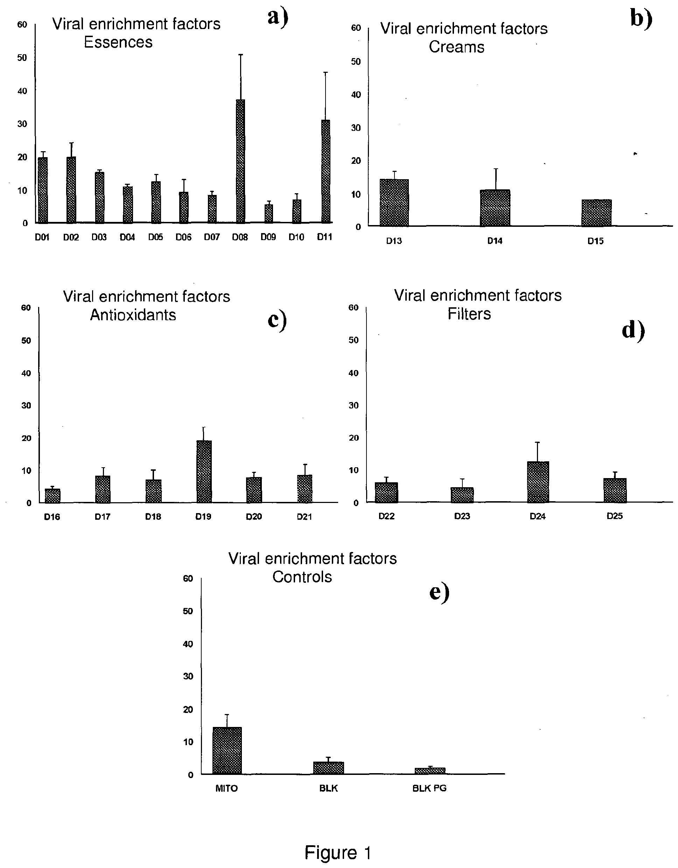

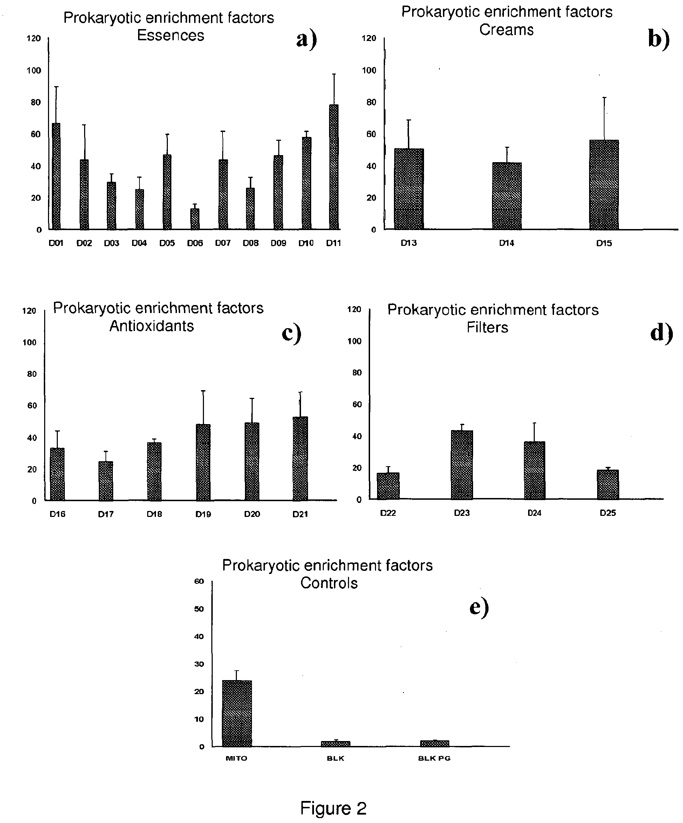

Viral abundances significantly increased of about 1 order of magnitude during the incubation experiments. Viral enrichment ranged from 1.9 to 37.4, in the systems BLK PG and D08 (Figure 1). Viral abundances at the end of the experiment ranged from 0.44 to 31.2 x 10⁵ viruses ml⁻¹ in systems BLK PG and D15, respectively. Prokaryotic abundances increased of 1-2 orders of magnitude during the incubation experiment. Prokaryiotes enrichment factors fluctuated within a wide range (Figure 2). The lowest enrichment factor (1.88) was observed in the BLK PG system, whereas in system D11 the enrichment factor was 78.16. A slight reduction of ratios between viral and prokaryotic abundance (VPR) after incubations was observed in all systems, with the exception of systems D07 and BLK, where VPR remained stable over time. VPR decreased up to 10 times in system D22.

### 4.2. Zooxanthellae abundance

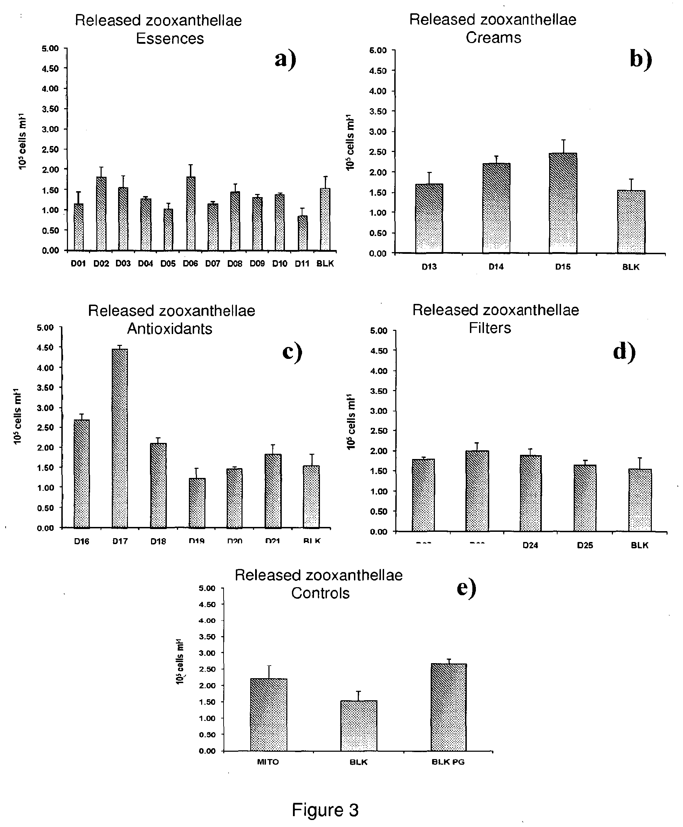

At the start of the incubations, in the microcosms no released zooxanthellae were found. The number of zooxanthellae increased over time in all microcosms considered. The final abundance of zooxanthellae found in water surrounding the coral nubbins ranged from 0.85 to 4.45 x 10⁴ cell ml⁻¹, respectively in systems D11 (inoculated with Joyful 60029MC) and D18 (inoculated with 2-phospho-L-ascorbic acid trisodium salt), (Figure 3a, b, c, d, e).

In some cases, abundance of released zooxanthellae was lower in systems treated than in controls. Statistical analyses revealed that only in the case of systems D15, D16, D17 and D18 the abundance of released cells was significantly higher than in the BLK system (P<0.01). For all other compounds no significant differences were found between control and treated systems, suggesting that the presence of the compounds tested in the experimental systems (with the exception of D15, D16, D17 and D18) causes no increase in the rate of zooxanthellae release from corals.

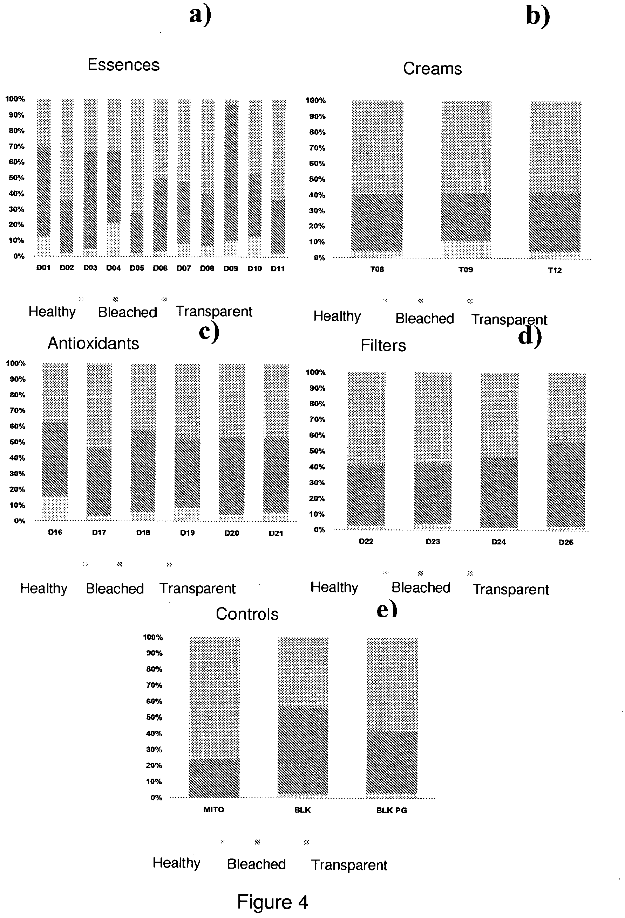

During microscopic analyses, observed zooxanthellae were split into three main groups (healthy, pale and transparent) as described in the Materials and methods section. At the end of the incubations, each group exhibited different abundances in the water surrounding the coral nubbins (Figure 4). Pale and transparent zooxanthellae were, in all experimental systems, the dominant groups, on average representing 44% and 50% of total released zooxanthellae. Healthy zooxanthellae always represented the lesser fraction, ranging from 0 to 21%.

The low abundance of healthy cells suggests that the incubation time of the experiments causes a high level of damage to zooxanthellae in all systems considered, negative controls (BLK) included.

### 4.3. Coral bleaching

In all replicates of every microcosm (control systems included), the release of coral mucous (comprised of zooxanthellae and coral tissue) was observed within the first 48 h of incubation. This observation is in accordance with the results of the release of zooxanthellae reported in section 4.2. Mucous release by coral fragments could be related to the mechanical stress occurred during sampling and preparation of microcosms. In all microcosms (BLK systems included) after 72 h of incubation a slight change in color was observed in coral nubbins, potentially occurred as a consequence of the stress induced by incubations. The most evident changes were observed in systems D18 and MITO, where all 4 color channels were significantly altered at the end of the incubations. In systems D05, D08, and BLK PG, 3 out of 4 color channels showed alterations. In all other systems, 2 or 1 color channels showed significant changes. The systems D03, D09, D10, D11, D21, D21 showed changes in 2 color channels, whereas systems D01, D04, D05, D13, D14, D19, D20 and BLK showed changes only in 1 color channel. In all other experimental systems, no color changes were detected in tested nubbins.

### 4.4 Photostability of the sunscreen cream exposed to UVA rays (∼ 275 kJ/m²)

2 mg cm⁻² of cream were spread on glass slides (5 x 5 cm) and exposed to UVA rays.
• UVA absorbance, 320-400 nm (peak corresponding to the DHHB filter) is too high with respect to the UVB one, 290-320 nm (peak corresponding to the EHT filter). UVA absorbance should be about 1/3 of the UVB one.
• The spectrum of the wide-spectrum filter, MBBT, is not visible since it has an extinction coefficient lower than DHHB and EHT, therefore is covered by the absorbance of the latter. MBBT should appear as a wide spectrum without maximum and minimum peaks.
• At the moment, obtained absorbance falls within the range of a sunscreen cream with a high and low SPF.
• According to the results, it can be concluded that the filters are highly photostable.

### 4.5 Summary of criteria used to test eco-compatibility of tested products with coral reefs:

In order to select the most eco-compatible products for each group of compounds (i.e., base cream, fragrance, antioxidants and filters) the following criteria were selected:
1. coral bleaching level;
2. degree of release of symbiotic zooxanthellae;
3. zooxanthellae health conditions;
4. viral enrichment in the incubation medium with respect to controls.

**Table 4. Summary of eco-compatible compounds grouped by category.**

| **Compound group** | **Ingredients** | **Eco-compatibility** |
|---|---|---|
| Base cream | Type 1 | Highly compatible |
| | Type 2 | Compatible |
| | Type 3 | Compatible |
| Fragrances | Guava & Coconut | Highly compatible |
| | Orange | Compatible |
| | Lavender | Compatible |
| | Grapefruit | Compatible |
| Antioxidants | Tocopheryl acetate | Highly compatible |
| | Tocopherol | Highly compatible |
| | Retinol | Compatible |
| | Polyphenone-60 | Compatible |
| | Retinyl palmitate | Compatible |
| UV filters | MBBT | Highly compatible |
| | DHHB | Highly compatible |
| | EHT | Compatible |

**Table 5. Viral abundance and enrichment during the incubations. Incubation times (Incub.), total viral abundances (TVN) and the viral enrichment factor (VEF) are also reported. Standard deviation values (STD) and variation coefficients are reported for all data.**

| **System** | **Incub.** | **TVN** | **STD** | **CV** | **VEF** | **STD** |
|---|---|---|---|---|---|---|
| D01 | 0 | 9.50E+04 | 2.69E+04 | 2.83E+01 | 19.62 | 2.00 |
| | 12 h | 1.83E+06 | 3.39E+05 | 1.85E+01 | | |
| D02 | 0 | 8.13E+04 | 2.11E+04 | 2.60E+01 | 19.87 | 4.43 |
| | 12 h | 1.57E+06 | 3.34E+05 | 2.12E+01 | | |
| D03 | 0 | 9.82E+04 | 2.19E+04 | 2.23E+01 | 15.48 | 0.81 |
| | 12 h | 1.51E+06 | 2.99E+05 | 1.98E+01 | | |
| D04 | 0 | 1.71E+05 | 3.08E+04 | 1.80E+01 | 11.01 | 0.80 |
| | 12 h | 1.87E+06 | 2.18E+05 | 1.17E+01 | | |
| D05 | 0 | 9.74E+04 | 1.94E+04 | 1.99E+01 | 12.44 | 2.28 |
| | 12 h | 1.20E+06 | 2.29E+05 | 1.92E+01 | | |
| D06 | 0 | 1.14E+05 | 2.67E+04 | 2.34E+01 | 9.37 | 4.06 |
| | 12 h | 1.02E+06 | 3.88E+05 | 3.79E+01 | | |
| D07 | 0 | 2.81E+05 | 2.48E+04 | 8.82E+00 | 8.50 | 1.08 |
| | 12 h | 2.38E+06 | 3.09E+05 | 1.30E+01 | | |
| D08 | 0 | 7.49E+04 | 2.30E+04 | 3.08E+01 | 37.39 | 13.49 |
| | 12 h | 2.60E+06 | 1.45E+05 | 5.58E+00 | | |
| D09 | 0 | 3.56E+05 | 6.28E+04 | 1.77E+01 | 5.55 | 0.93 |
| | 12 h | 1.94E+06 | 1.96E+05 | 1.01E+01 | | |
| D10 | 0 | 2.20E+05 | 5.34E+04 | 2.43E+01 | 7.18 | 1.80 |
| | 12 h | 1.52E+06 | 1.31E+05 | 8.58E+00 | | |
| D11 | 0 | 1.14E+05 | 5.77E+04 | 5.08E+01 | 30.89 | 14.56 |
| | 12 h | 2.95E+06 | 5.30E+05 | 1.80E+01 | | |
| D13 | 0 | 1.34E+05 | 3.42E+04 | 2.56E+01 | 14.29 | 2.60 |
| | 12 h | 1.87E+06 | 4.29E+05 | 2.29E+01 | | |
| D14 | 0 | 2.35E+05 | 5.20E+04 | 2.21E+01 | 11.03 | 6.65 |
| | 12 h | 2.36E+06 | 1.03E+06 | 4.35E+01 | | |
| D15 | 0 | 3.79E+05 | 8.90E+04 | 2.35E+01 | 8.21 | 0.28 |
| | 12 h | 3.12E+06 | 7.64E+05 | 2.45E+01 | | |
| D16 | 0 | 3.24E+05 | 7.97E+04 | 2.46E+01 | 4.54 | 0.48 |
| | 12 h | 1.46E+06 | 2.87E+05 | 1.97E+01 | | |
| D17 | 0 | 3.49E+05 | 6.79E+04 | 1.94E+01 | 8.36 | 2.46 |
| | 12 h | 2.83E+06 | 6.23E+05 | 2.20E+01 | | |
| D18 | 0 | 3.70E+05 | 5.71E+04 | 1.55E+01 | 7.09 | 3.00 |
| | 12 h | 2.51E+06 | 6.73E+05 | 2.69E+01 | | |
| D19 | 0 | 1.03E+05 | 1.37E+04 | 1.33E+01 | 18.92 | 4.43 |
| | 12 h | 1.98E+06 | 6.60E+05 | 3.33E+01 | | |
| D20 | 0 | 2.47E+05 | 7.76E+04 | 3.14E+01 | 8.09 | 1.18 |
| | 12 h | 1.97E+06 | 4.92E+05 | 2.50E+01 | | |
| D21 | 0 | 3.23E+05 | 9.01E+04 | 2.79E+01 | 8.56 | 3.32 |
| | 12 h | 2.57E+06 | 1.94E+05 | 7.54E+00 | | |
| D22 | 0 | 5.06E+05 | 1.12E+05 | 2.21E+01 | 6.16 | 1.77 |
| | 12 h | 2.99E+06 | 3.50E+05 | 1.17E+01 | | |
| D23 | 0 | 1.87E+05 | 3.30E+04 | 1.77E+01 | 4.80 | 2.60 |
| | 12 h | 8.65E+05 | 3.77E+05 | 4.36E+01 | | |
| D24 | 0 | 9.58E+04 | 3.38E+04 | 3.53E+01 | 12.58 | 5.97 |
| | 12 h | 1.08E+06 | 1.96E+05 | 1.81E+01 | | |
| D25 | 0 | 3.67E+05 | 8.46E+04 | 2.30E+01 | 7.44 | 1.79 |
| | 12 h | 2.63E+06 | 8.45E+04 | 3.21E+00 | | |
| MITO | 0 | 1.11E+05 | 2.20E+04 | 1.98E+01 | 14.33 | 4.09 |
| | 12 h | 1.53E+06 | 1.45E+05 | 9.46E+00 | | |
| BLK | 0 | 2.03E+04 | 3.61E+03 | 1.78E+01 | 3.68 | 1.62 |
| | 12 h | 4.45E+04 | 4.01E+04 | 9.00E+01 | | |
| BLK PG | 0 | 2.42E+04 | 4.26E+03 | 1.76E+01 | 1.88 | 0.64 |
| | 12 h | 4.37E+04 | 8.06E+03 | 1.84E+01 | | |

**Table 6. Bacterial abundance (total bacterial number, TBN) during incubations, and related standard deviations and coefficient of variation.**

| **System** | **Incubation** | **TBN** | **STD** | **CV** |
|---|---|---|---|---|
| D01 | 0 | 1.93E+04 | 3.42E+03 | 1.77E+01 |
| | 12 h | 1.21E+06 | 1.66E+05 | 1.37E+01 |
| D02 | 0 | 2.17E+04 | 6.39E+03 | 2.94E+01 |
| | 12 h | 8.48E+05 | 1.42E+05 | 1.67E+01 |
| D03 | 0 | 3.14E+04 | 4.83E+03 | 1.54E+01 |
| | 12 h | 9.20E+05 | 1.04E+05 | 1.13E+01 |
| D04 | 0 | 3.54E+04 | 1.09E+04 | 3.07E+01 |
| | 12 h | 8.43E+05 | 9.84E+04 | 1.17E+01 |
| D05 | 0 | 7.25E+03 | 2.42E+03 | 3.33E+01 |
| | 12 h | 3.19E+05 | 2.59E+04 | 8.10E+00 |
| D06 | 0 | 3.94E+04 | 5.03E+03 | 1.27E+01 |
| | 12 h | 4.99E+05 | 5.03E+04 | 1.01E+01 |
| D07 | 0 | 2.74E+04 | 7.76E+03 | 2.84E+01 |
| | 12 h | 1.11E+06 | 2.42E+05 | 2.18E+01 |
| D08 | 0 | 4.75E+04 | 2.79E+03 | 5.87E+00 |
| | 12 h | 1.22E+06 | 2.74E+05 | 2.25E+01 |
| D09 | 0 | 2.58E+04 | 1.39E+03 | 5.41E+00 |
| | 12 h | 1.18E+06 | 1.87E+05 | 1.58E+01 |
| D10 | 0 | 1.69E+04 | 2.42E+03 | 1.43E+01 |
| | 12 h | 9.74E+05 | 1.12E+05 | 1.15E+01 |
| D11 | 0 | 2.50E+04 | 6.08E+03 | 2.44E+01 |
| | 12 h | 1.88E+06 | 2.83E+05 | 1.50E+01 |
| D13 | 0 | 2.82E+04 | 6.08E+03 | 2.16E+01 |
| | 12 h | 1.35E+06 | 1.73E+05 | 1.28E+01 |
| D14 | 0 | 4.67E+04 | 3.69E+03 | 7.90E+00 |
| | 12 h | 1.92E+06 | 3.52E+05 | 1.83E+01 |
| D15 | 0 | 2.01E+04 | 5.03E+03 | 2.50E+01 |
| | 12 h | 1.05E+06 | 2.11E+05 | 2.02E+01 |
| D16 | 0 | 3.78E+04 | 1.01E+04 | 2.66E+01 |
| | 12 h | 1.18E+06 | 2.51E+04 | 2.13E+00 |
| D17 | 0 | 5.80E+04 | 1.05E+04 | 1.82E+01 |
| | 12 h | 1.42E+06 | 3.82E+05 | 2.70E+01 |
| D18 | 0 | 3.86E+04 | 6.39E+03 | 1.65E+01 |
| | 12 h | 1.39E+06 | 1.22E+05 | 8.73E+00 |
| D19 | 0 | 3.62E+04 | 1.45E+04 | 4.00E+01 |
| | 12 h | 1.54E+06 | 1.58E+05 | 1.02E+01 |
| D20 | 0 | 3.62E+04 | 9.66E+03 | 2.67E+01 |
| | 12 h | 1.71E+06 | 4.32E+05 | 2.53E+01 |
| D21 | 0 | 3.06E+04 | 6.08E+03 | 1.99E+01 |
| | 12 h | 1.57E+06 | 1.99E+05 | 1.27E+01 |
| D22 | 0 | 5.47E+04 | 9.14E+03 | 1.67E+01 |
| | 12 h | 9.18E+05 | 2.90E+05 | 3.16E+01 |
| D23 | 0 | 4.67E+04 | 3.69E+03 | 7.90E+00 |
| | 12 h | 2.03E+06 | 3.31E+05 | 1.63E+01 |
| D24 | 0 | 2.09E+04 | 3.69E+03 | 1.76E+01 |
| | 12 h | 7.41E+05 | 2.13E+05 | 2.87E+01 |
| D25 | 0 | 3.67E+05 | 8.46E+04 | 2.30E+01 |
| | 12 h | 2.63E+06 | 8.45E+04 | 3.21E+00 |
| MITO | 0 | 1.11E+05 | 2.20E+04 | 1.98E+01 |
| | 12 h | 1.53E+06 | 1.45E+05 | 9.46E+00 |
| BLK | 0 | 2.03E+04 | 3.61E+03 | 1.78E+01 |
| | 12 h | 4.45E+04 | 4.01E+04 | 9.00E+01 |
| BLK PG | 0 | 2.42E+04 | 4.26E+03 | 1.76E+01 |
| | 12 h | 4.37E+04 | 8.06E+03 | 1.84E+01 |

**Table 7. Number of healthy, pale and transparent (i.e., dead) zooxanthellae (in cells ml⁻¹) released in each microcosm.**

| **Systems** | **Total** | **STD** | **CV** | **Healthy** | **STD** | **CV** | **Pale** | **STD** | **CV** | **Transparent** | **STD** | **CV** |
|---|---|---|---|---|---|---|---|---|---|---|---|---|
| D01 | 1.16E+04 | 2.93E+03 | 25.3 | 1.41E+03 | 9.22E+02 | 65.5 | 6.78E+03 | 9.51E+02 | 14.0 | 3.42E+03 | 1.07E+03 | 31.1 |
| D02 | 1.81E+04 | 2.51E+03 | 13.9 | 3.35E+02 | 1.16E+02 | 34.6 | 6.11E+03 | 4.19E+02 | 6.9 | 1.17E+04 | 2.82E+03 | 24.1 |
| D03 | 1.56E+04 | 2.92E+03 | 18.7 | 7.04E+02 | 1.42E+02 | 20.2 | 9.73E+03 | 2.62 E+03 | 26.9 | 5.43E+03 | 2.01E+02 | 3.7 |
| D04 | 1.28E+04 | 5.81 E+02 | 4.5 | 2.68E+03 | 9.08E+02 | 33.8 | 5.90E+03 | 1.11E+03 | 18.8 | 4.23E+03 | 1.22E+03 | 29.0 |
| D05 | 1.03E+04 | 1.45 E+03 | 14.1 | 2.01E+02 | 0.00E+00 | 0.0 | 2.62E+03 | 7.26E+02 | 27.7 | 7.51E+03 | 8.13E+02 | 10.8 |
| D06 | 1.81E+04 | 3.12E+03 | 17.2 | 6.04E+02 | 4.03E+02 | 66.7 | 8.45E+03 | 1.84E+03 | 21.8 | 9.06E+03 | 1.22E+03 | 13.5 |
| D07 | 1.15E+04 | 7.26E+02 | 6.3 | 8.72E+02 | 2.32E+02 | 26.6 | 4.63E+03 | 1.01E+03 | 21.7 | 5.97E+03 | 3.07E+02 | 5.1 |
| D08 | 1.44E+04 | 2.13E+03 | 14.8 | 9.39E+02 | 2.32E+02 | 24.7 | 4.90E+03 | 7.07E+02 | 14.4 | 8.59E+03 | 1.21E+03 | 14.1 |
| D09 | 1.31E+04 | 6.97E+02 | 5.3 | 1.34E+03 | 3.07E+02 | 22.9 | 1.18E+04 | 1.80E+03 | 15.3 | 4.03E+02 | O.OOE+00 | 0.0 |
| D10 | 1.38E+04 | 4.65E+02 | 3.4 | 1.74E+03 | 3.07E+02 | 17.6 | 5.43 E+03 | 1.01E+03 | 18.5 | 6.57E+03 | 8.13E+02 | 12.4 |
| D11 | 8.45E+03 | 1.98E+03 | 23.4 | 2.01E+02 | 0.00E+00 | 0.0 | 2.88E+03 | 1.23E+03 | 42.6 | 5.43E+03 | 7.26E+02 | 13.4 |
| D12 | 1.18E+04 | 6.47E+02 | 5.5 | 6.71E+01 | 1.16E+02 | 173.2 | 5.64E+03 | 7.26E+02 | 12.9 | 6.11E+03 | 6.47E+02 | 10.6 |
| D13 | 1.71E+04 | 2.82E+03 | 16.5 | 6.71E+02 | 3.07E+02 | 45.8 | 6.31E+03 | 1.83E+03 | 29.0 | 1.01E+04 | 1.37E+03 | 13.5 |
| D14 | 2.22E+04 | 1.82E+03 | 8.2 | 2.42E+03 | 6.04E+02 | 25.0 | 6.84E+03 | 1.07E+03 | 15.6 | 1.29E+04 | 1.31E+03 | 10.1 |
| D15 | 2.48E+04 | 3.39E+03 | 13.7 | 1.07E+03 | 3.07E+02 | 28.6 | 9.33E+03 | 1.86E+03 | 19.9 | 1.44E+04 | 2.62E+03 | 18.2 |
| D16 | 2.67E+04 | 1.74E+03 | 6.5 | 4.09E+03 | 6.47E+02 | 15.8 | 1.25E+04 | 1.91E+03 | 15.2 | 1.01E+04 | 4.03E+02 | 4.0 |
| D17 | 4.45E+04 | 8.77E+02 | 2.0 | 1.48E+03 | 4.19E+02 | 28.4 | 1.88E+04 | 1.56E+03 | 8.3 | 2.42E+04 | 1.98E+03 | 8.2 |
| D18 | 2.11E+04 | 1.34E+03 | 6.4 | 1.14E+03 | 4.19E+02 | 36.7 | 1.09E+04 | 1.60E+03 | 14.7 | 9.06E+03 | 1.94E+03 | 21.4 |
| D19 | 1.23E+04 | 2.40E+03 | 19.5 | 1.07E+03 | 1.16E+02 | 10.8 | 5.30E+03 | 1.14E+03 | 21.6 | 5.97E+03 | 1.23E+03 | 20.6 |
| D20 | 1.46E+04 | 6.47E+02 | 4.4 | 6.04E+02 | 2.01E+02 | 33.3 | 7.18E+03 | 1.23E+03 | 17.1 | 6.84E+03 | 9.22E+02 | 13.5 |
| D21 | 1.83E+04 | 2.29E+03 | 12.5 | 1.07E+03 | 3.07E+02 | 28.6 | 8.59E+03 | 1.48E+03 | 17.3 | 8.65E+03 | 5.33E+02 | 6.2 |
| D22 | 1.80E+04 | 6.15E+02 | 3.4 | 4.70E+02 | 1.16E+02 | 24.7 | 6.91E+03 | 5.81E+02 | 8.4 | 1.06E+04 | 1.14E+03 | 10.8 |
| D23 | 2.00E+04 | 2.03E+03 | 10.1 | 7.38E+02 | 1.16E+02 | 15.7 | 7.58E+03 | 6.15E+02 | 8.1 | 1.17E+04 | 2.62E+03 | 22.4 |
| D24 | 1.89E+04 | 1.69E+03 | 9.0 | 2.68E+02 | 1.16E+02 | 43.3 | 8.39E+03 | 6.15 E+02 | 7.3 | 1.02E+04 | 1.23E+03 | 12.1 |
| D25 | 1.64E+04 | 1.31E+03 | 8.0 | 3.35E+02 | 1.16E+02 | 34.6 | 8.86E+03 | 1.57E+03 | 17.8 | 7.25E+03 | 7.26E+02 | 10.0 |
| MITO | 2.20E+04 | 4.22E+03 | 19.2 | 0.00E+00 | 0.00E+00 | 0.0 | 5.25E+03 | 4.33E+02 | 8.2 | 1.68E+04 | 3.88E+03 | 23.2 |
| BLK | 1.64E+04 | 1.31E+03 | 8.0 | 3.35E+02 | 1.16E+02 | 34.6 | 8.86E+03 | 1.57E+03 | 17.8 | 7.25E+03 | 7.26E+02 | 10.0 |
| BLK PG | 2.66E+04 | 1.54E+03 | 5.8 | 8.05E+02 | 2.01E+02 | 25.0 | 1.03E+04 | 8.13E+02 | 7.9 | 1.55E+04 | 8.77E+02 | 5.7 |

**Table 8. Percentage of healthy, pale and transparent zooxanthellae released in the microcosms.**

| **Systems** | **Total** | **Healthy** | **Pale** | **Trasparent** |
|---|---|---|---|---|
| D01 | 100 | 12 | 58 | 29 |
| D02 | 100 | 2 | 34 | 64 |
| D03 | 100 | 5 | 62 | 35 |
| D04 | 100 | 21 | 46 | 33 |
| D05 | 100 | 2 | 25 | 73 |
| D06 | 100 | 3 | 47 | 50 |
| D07 | 100 | 8 | 40 | 52 |
| D08 | 100 | 7 | 34 | 60 |
| D09 | 100 | 10 | 90 | 3 |
| D10 | 100 | 13 | 40 | 48 |
| D11 | 100 | 2 | 34 | 64 |
| D13 | 100 | 4 | 37 | 59 |
| D14 | 100 | 11 | 31 | 58 |
| D15 | 100 | 4 | 38 | 58 |
| D16 | 100 | 15 | 47 | 38 |
| D17 | 100 | 3 | 42 | 54 |
| D18 | 100 | 5 | 52 | 43 |
| D19 | 100 | 9 | 43 | 48 |
| D20 | 100 | 4 | 49 | 47 |
| D21 | 100 | 6 | 47 | 47 |
| D22 | 100 | 3 | 38 | 59 |
| D23 | 100 | 4 | 38 | 58 |
| D24 | 100 | 1 | 44 | 54 |
| D25 | 100 | 2 | 54 | 44 |
| MITO | 100 | 0 | 24 | 76 |
| BLK | 100 | 2 | 54 | 44 |
| BLKPG | 100 | 3 | 39 | 58 |

**Table 9. Output of one-way ANOVA analysis for the different color components of corals. Probability level reported (**=P<0.01; *=P<0.05; NS = not significant).**

| **Sample code** | **Cyan (C)** | **Magenta (M)** | **Yellow (Y)** | **Black (K)** |
|---|---|---|---|---|
| D01 | NS | NS | NS | * |
| D02 | NS | * | NS | NS |
| D03 | NS | * | NS | * |
| D04 | NS | ** | NS | NS |
| D05 | * | NS | NS | NS |
| D06 | * | ** | NS | ** |
| D07 | NS | NS | NS | NS |
| D08 | * | ** | NS | ** |
| D09 | * | NS | NS | * |
| D10 | NS | ** | NS | ** |
| D11 | NS | NS | * | * |
| D13 | ** | NS | NS | NS |
| D14 | NS | NS | NS | * |
| D15 | NS | NS | NS | NS |
| D16 | NS | NS | NS | NS |
| D17 | NS | NS | NS | NS |
| D18 | ** | ** | * | ** |
| D19 | NS | * | NS | NS |
| D20 | * | NS | NS | NS |
| D21 | NS | NS | NS | ** |
| D22 | NS | NS | NS | NS |
| D23 | ** | NS | * | NS |
| D24 | NS | NS | NS | NS |
| D25 | NS | NS | NS | NS |
| BLK | NS | * | NS | NS |
| BLK PG | ** | ** | NS | ** |
| MITO | ** | ** | ** | ** |

### REFERENCE

Balmer M.E., Buser H.R., Muller M.D., Poiger T. (2005). Occurrence of some organic UV filters in wastewater, in surface waters, and in fish from Swiss lakes. Environmental Science Technology, 39: 953-962.
Brown B.E., Dunne R.P., Goodson M.S., Douglas A.E. (2000). Marine ecology: bleaching patterns in reef corals. Nature, 404: 142-143.
Bruno J.F., Selig E.R., Casey K.S., Page C.A., Willis B.L., Harvell C.D. (2007). Thermal stress and coral cover as drivers of coral disease outbreaks. PLoS Biology. 5: 1220-1227.
Clarke K.R. & Gorley R.N. (2001). PRIMER v5:User manual/tutorial, PRIMER-E, Plymouth UK, 91pp.
Connell, J.H., Hughes T.P., Wallace C.C. (1997). A 30-year study of coral abundance, recruitment and disturbance at several scales in space and time. Ecological Monographs, 67: 461-488.
Danovaro R. & Corinaldesi C. (2003). Sunscreen products increase virus production through prophage induction in marine bacterioplankton. Microbial Ecology. 45: 109-118.
Danovaro R., Bongiorni L., Corinaldesi C., Giovannelli D., Damiani E., Astolfi P., Greci L., Pusceddu A. (2008). Sunscreens cause coral bleaching by promoting viral infections. Environmental Health Perspectives. 116: 441-447.
Daughton C.G. & Ternes T.A. (1999). Pharmaceuticals and personal care products in the environment: agents of subtle change? Environmental Health Perspective. 107: 907-938.
Douglas A.E. (2003). Coral bleaching-how and why? Marine Pollution Bulletin. 46: 385-392.
Giokas D.L., Salvador A., Chisvert A. (2007). UV filters: from sunscreens to human body and the environment. Trends in Analytical Chemistry. 26: 360-374.
Glynn P.W.(1996). Coral reef bleaching: facts, hypotheses and implications. Global Change Biology. 2: 495-509.
Hoegh-Guldberg O. (1999). Climate change, coral bleaching and the future of the world's coral reefs. Marine Freshwater Research. 50: 839-866.
Hughes T.P., Baird A.H., Bellwood D.R., Card M., Connolly S.R. (2003). Climate change, human impacts, and the resilience of coral reefs. Science. 301: 929-933. Jones R.J. (2004). Testing the 'photoinhibition' model of coral bleaching using chemical inhibitors. Marine Ecology Progress Series. 284: 133-145.
Knowlton N. (2001). The future of coral reefs. Proceedings of the National Academy of Sciences of USA. 98: 5419-5425.
Mise T. & Hidaka M. (2003). Degradation of zooxanthellae in the coral Acropora nasuta during bleaching. Galaxea JCRS. 5: 33-39.
Moberg F. & Folke C. (1999). Ecological goods and services of coral reef ecosystems. Ecological Economics. 29: 215-233.
Pandolfi J.M., Bradbury R.H., Sala E., Hughes T.P., Bjorndal K.A., Cooke R.G. (2003). Global trajectories of the long term decline of coral reef ecosystems. Science. 301: 955-958.
Schlumpf M., Schmid P., Durrer S., Conscience M., Maerkel K. (2004). Endocrine activity and developmental toxicity of cosmetic UV filters-an update. Toxicology. 205: 113-122.
Shibata A., Goto Y., Saito H., Kikuchi T., Toda T., Taguchi S. (2006). Comparison of SYBR Green I and SYBR Gold stains for enumerating bacteria and viruses by epifluorescence microscopy. Aquatic Microbial Ecology: 43: 223-231.
Suharsono (1992). Coral assemblages around Palau Genteng Besar, Seribu Islands, Indonesia. In L. M. Chou and C.R. Wilkinson (eds.), 3rd ASEAN Science and Technology Week Conference Proceedings, Vol. 6, Marine Science: Living Coastal Resources, 21-23 Sept. 1992, Singapore, National University of Singapore. Pp.41-54. Siebeck U.E., Marshall N.J., Klüter A., Hoegh-Guldberg O. (2006). Monitoring coral bleaching using a colour reference card. Coral Reefs. 25: 453-460.
Veron J.E.N., Devantier L.M., Turak E., Green A. L., Kininmonth S., Stafford-Smith S., Peterson N. (2009). Delineating the Coral Triangle. Galaxea, Journal of Coral Reef Studies 11: 91-100.
Wallace, C.C. (1999). Staghorn Corals of the World: A revision of the coral genus Acropora (Scleractinia; Astrocoeniina; Acroporidae) worldwide, with emphasis on morphology, phylogeny and biogeography, Pp. 422. Melbourne: CSIRO Publishing. Wallace C.C., Richards Z., Sharshono (2001). Regional distribution patterns of Acropora and their use in the conservation of coral reefs in Indonesia.
Wilkinson C. (2004). Status of Coral Reefs of the World: 2004. Townsville, Australia: Australian Institute for Marine Science.

## Claims

1. An eco-compatible sunscreen composition comprising:
- the UV filters 2,2'-methylene-bis-(6-(2H-benzotriazole-2-yl)-4-(1,1,3,3-tetramethylbutyl)-phenol, 2-[4-(diethylamine)-2-hydroxybenzoyl]hexyl benzoate and ethylhexyl triazone;
- one or more antioxidants selected from tocopheryl acetate, tocopherol, retinol, polyphenolic compounds extracted from green tea;
- one or more carriers selected from propylene glycol dicaprylate/dicaprate, olive oil, beeswax. and free from:
- preservatives belonging to the paraben family;
- ascorbic acid derivatives;
- UV filters selected from bis ethylhexyloxyphenol methoxyphenyl triazine, cinnamates, benzophenones, camphor derivatives, titanium oxide;
- fragrances selected from melaleuca essential oil, ylang ylang flower extracts, Bambousa Arundinacea extracts, Karitè;
- carriers selected from animal-derived fats and argan oil.

2. An eco-compatible sunscreen composition comprising:
- one or more UV filters selected from 2,2'-methylene-bis-(6-(2H-benzotriazole-2-yl)-4-(1,1,3,3-tetramethylbutyl)-phenol, 2-[4-(diethylamine)-2-hydroxybenzoyl]hexyl benzoate, ethylhexyl triazone;
- one or more antioxidants selected from tocopheryl acetate, tocopherol, retinol, polyphenolic compounds extracted from green tea;
- one or more carriers selected from propylene glycol dicaprylate/dicaprate, olive oil, beeswax,
- and further comprising one or more fragrances selected from orange, lavender, grapefruit, guava and coconut.
and free from:
- preservatives belonging to the paraben family;
- ascorbic acid derivatives;
- UV filters selected from bis ethylhexyloxyphenol methoxyphenyl triazine, cinnamates, benzophenones, camphor derivatives, titanium oxide;
- fragrances selected from melaleuca essential oil, ylang ylang flower extracts, Bambousa Arundinacea extracts, Karitè;
- carriers selected from animal-derived fats and argan oil.

3. An eco-compatible sunscreen composition comprising:
- one or more UV filters selected from 2,2'-methylene-bis-(6-(2H-benzotriazole-2-yl)-4-(1,1,3,3-tetramethylbutyl)-phenol, 2-[4-(diethylamine)-2-hydroxybenzoyl]hexyl benzoate, ethylhexyl triazone;
- one or more antioxidants selected from tocopheryl acetate, tocopherol, retinol, polyphenolic compounds extracted from green tea;
- one or more carriers selected from propylene glycol dicaprylate/dicaprate, olive oil, beeswax, further comprising sorbic acid
and free from:
- preservatives belonging to the paraben family;
- ascorbic acid derivatives;
- UV filters selected from bis ethylhexyloxyphenol methoxyphenyl triazine, cinnamates, benzophenones, camphor derivatives, titanium oxide;
- fragrances selected from melaleuca essential oil, ylang ylang flower extracts, Bambousa Arundinacea extracts, Karitè;
- carriers selected from animal-derived fats and argan oil

4. The composition according to claim 1 further comprising one or more fragrances selected from orange, lavender, grapefruit, guava and coconut and/or sorbic acid.

5. The composition according to claim 2 further comprising sorbic acid.

6. The composition according to claim 1, consisting of:
- 2,2'-methylene-bis-(6-(2H-benzotriazole-2-yl)-4-(1,1,3,3-tetramethylbutyl)-phenol, 2-[4-(diethylamine)-2-hydroxybenzoyl]hexyl benzoate and ethylhexyl triazone;
- one or more antioxidants selected from tocopheryl acetate, tocopherol, retinol, polyphenolic compounds extracted from green tea;
- one or more carriers selected from propylene glycol dicaprylate/dicaprate, olive oil, beeswax;
- water.

7. The composition according to claim 1, consisting of:
- 2,2'-methylene-bis-(6-(2H-benzotriazole-2-yl)-4-(1,1,3,3-tetramethylbutyl)-phenol, 2-[4-(diethylamine)-2-hydroxybenzoyl]hexyl benzoate and ethylhexyl triazone;
- one or more antioxidants selected from tocopheryl acetate, tocopherol, retinol, polyphenolic compounds extracted from green tea;
- sorbic acid;
- one or more carriers selected from propylene glycol dicaprylate/dicaprate, olive oil, beeswax;
- water.

8. The composition according to claim 1, consisting of:
- 2,2'-methylene-bis-(6-(2H-benzotriazole-2-yl)-4-(1,1,3,3-tetramethylbutyl)-phenol, 2-[4-(diethylamine)-2-hydroxybenzoyl]hexyl benzoate and ethylhexyl triazone;
- tocopheryl acetate;
- sorbic acid;
- propylene glycol dicaprylate/dicaprate, olive oil and beeswax;
- water;
- hydroxyethyl cellulose;
- EDTA;
- sodium hydroxide.

9. The composition according to any one of the claims 1 to 6, wherein said UV filters are present in a concentration comprised between 3 and 6% by weight.

10. The composition according to any one of the claims 1 to 9, in the form of cream, lotion, ointment, spray, lipstick.

11. An eco-compatible sunscreen composition comprising:
- one or more UV filters selected from 2,2'-methylene-bis-(6-(2H-benzotriazole-2-yl)-4-(1,1,3,3-tetramethylbutyl)-phenol, 2-[4-(diethylamine)-2-hydroxybenzoyl]hexyl benzoate, ethylhexyl triazone;
- one or more antioxidants selected from tocopherol, retinol, polyphenolic compounds extracted from green tea;
- one or more carriers selected from propylene glycol dicaprylate/dicaprate, olive oil, beeswax, and free from:
- preservatives belonging to the paraben family;
- ascorbic acid derivatives;
- UV filters selected from bis ethylhexyloxyphenol methoxyphenyl triazine, cinnamates, benzophenones, camphor derivatives, titanium oxide;
- fragrances selected from melaleuca essential oil, ylang ylang flower extracts, Bambousa Arundinacea extracts, Karitè;
- carriers selected from animal-derived fats and argan oil as a sunscreen not harmful to the coral reef.

## Patentansprüche

1. Eine bio-kompatible Sonnenschutzzusammensetzung, umfassend:
- die UV-Filter 2,2'-Methylen-bis-(6-(2H-benzotriazol-2-yl)-4-(1,1,3,3-tetramethylbutyl)-phenol, 2-[4-(Diethylamin)-2-hydroxybenzoyl]hexylbenzoat und Ethylhexyltriazon;
- ein oder mehrere Antioxidationsmittel, ausgewählt aus Tocopherylacetat, Tocopherol, Retinol, polyphenolischen Verbindungen, die von grünem Tee extrahiert sind;
- einen oder mehrere Träger, ausgewählt aus Propylenglykol-Dicaprylat/Dicaprat, Olivenöl, Bienenwachs;
und frei von:
- Konservierungsmitteln, die zur Paraben-Familie gehören;
- Ascorbinsäurederivaten;
- UV-Filtern, ausgewählt aus Bis-Ethylhexyloxyphenol-Methoxyphenyltriazin, Cinnamaten, Benzophenonen, Campherderivaten, Titaniumoxid;
- Duftstoffen, ausgewählt aus ätherischem Melaleuca-Öl, Ylang-Ylang-Blütenextrakten, Bambousa Arundinacea-Extrakten, Karitè;
- Trägern, ausgewählt aus Fetten tierischer Herkunft und Arganöl.

2. Eine bio-kompatible Sonnenschutzzusammensetzung, umfassend:
- einen oder mehrere UV-Filter, ausgewählt aus 2,2'-Methylen-bis-(6-(2H-Benzotriazol-2-yl)-4-(1,1,3,3-tetramethylbutyl)-phenol, 2-[4-(Diethylamin)-2-hydroxybenzoyl]hexylbenzoat, Ethylhexyltriazon;
- ein oder mehrere Antioxidationsmittel, ausgewählt aus Tocopherylacetat, Tocopherol, Retinol, polyphenolischen Verbindungen, die von grünem Tee extrahiert sind;
- einen oder mehrere Träger, ausgewählt aus Propylenglykol-Dicaprylat/Dicaprat, Olivenöl, Bienenwachs,
- und ferner umfassend einen oder mehrere Duftstoffe, ausgewählt aus Orange, Lavendel, Grapefruit, Guave und Kokosnuss;
und frei von:
- Konservierungsmitteln, die zur Paraben-Familie gehören;
- Ascorbinsäurederivaten;
- UV-Filtern, ausgewählt aus Bis-Ethylhexyloxyphenol-Methoxyphenyltriazin, Cinnamaten, Benzophenonen, Campherderivaten und Titanoxid;
- Duftstoffen, ausgewählt aus ätherischem Melaleuca-Öl, Ylang-Ylang-Blütenextrakten, Bambousa Arundinacea-Extrakten, Karitè;
- Trägern, ausgewählt aus Fetten tierischer Herkunft und Arganöl.

3. Eine bio-kompatible Sonnenschutzzusammensetzung, umfassend:
- einen oder mehrere UV-Filter, ausgewählt aus 2,2'-Methylen-bis-(6-(2H-Benzotriazol-2-yl)-4-(1,1,3,3-tetramethylbutyl)-phenol, 2-[4-(Diethylamin)-2-hydroxybenzoyl]hexylbenzoat, Ethylhexyltriazon;
- ein oder mehrere Antioxidationsmittel, ausgewählt aus Tocopherylacetat, Tocopherol, Retinol, polyphenolischen Verbindungen, die von grünem Tee extrahiert sind;
- einen oder mehrere Träger, ausgewählt aus Propylenglykol-Dicaprylat/Dicaprat, Olivenöl, Bienenwachs,
ferner umfassend Sorbinsäure
und frei von:
- Konservierungsmitteln, die zur Paraben-Familie gehören;
- Ascorbinsäurederivaten;
- UV-Filtern, ausgewählt aus Bis-Ethylhexyloxyphenol-Methoxyphenyltriazin, Cinnamaten, Benzophenonen, Campherderivaten, Titanoxid;
- Duftstoffen, ausgewählt aus ätherischem Melaleuca-Öl, Ylang-Ylang-Blütenextrakten, Bambousa Arundinacea-Extrakten, Karitè;
- Trägern, ausgewählt aus Fetten tierischer Herkunft und Arganöl

4. Zusammensetzung gemäß Anspruch 1 ferner umfassend einen oder mehrere Duftstoffe, ausgewählt aus Orange, Lavendel, Grapefruit, Guave und Kokosnuss und/oder Sorbinsäure.

5. Zusammensetzung gemäß Anspruch 2 ferner umfassend Sorbinsäure.

6. Zusammensetzung gemäß Anspruch 1, bestehend aus:
- 2,2'-Methylen-bis-(6-(2H-Benzotriazol-2-yl)-4-(1,1,3,3-tetramethylbutyl)-phenol, 2-[4-(Diethylamin)-2-hydroxybenzoyl]hexylbenzoat und Ethylhexyltriazon;
- einem oder mehreren Antioxidationsmitteln, ausgewählt aus Tocopherylacetat, Tocopherol, Retinol, polyphenolischen Verbindungen, die von grünem Tee extrahiert sind;
- einem oder mehreren Trägern, ausgewählt aus Propylenglykol-Dicaprylat/Dicaprat, Olivenöl, Bienenwachs;
- Wasser.

7. Zusammensetzung gemäß Anspruch 1, bestehend aus:
- 2,2'-Methylen-bis-(6-(2H-Benzotriazol-2-yl)-4-(1,1,3,3-tetramethylbutyl)-phenol, 2-[4-(Diethylamin)-2-hydroxybenzoyl]hexylbenzoat und Ethylhexyltriazon;
- einem oder mehreren Antioxidationsmitteln, ausgewählt aus Tocopherylacetat, Tocopherol, Retinol, polyphenolischen Verbindungen, die von grünem Tee extrahiert sind;
- Sorbinsäure;
- einem oder mehreren Trägern, ausgewählt aus Propylenglykol-Dicaprylat/Dicaprat, Olivenöl, Bienenwachs;
- Wasser.

8. Zusammensetzung gemäß Anspruch 1, bestehend aus:
- 2,2'-Methylen-bis-(6-(2H-Benzotriazol-2-yl)-4-(1,1,3,3-tetramethylbutyl)-phenol, 2-[4-(Diethylamin)-2-hydroxybenzoyl]hexylbenzoat und Ethylhexyltriazon;
- Tocopherylacetat;
- Sorbinsäure;
- Propylenglykol Dicaprylat/Dicaprat, Olivenöl und Bienenwachs;
- Wasser;
- Hydroxyethylcellulose;
- EDTA;
- Natriumhydroxid.

9. Zusammensetzung gemäß einem der Ansprüche 1 bis 6, wobei die UV-Filter in einer Konzentration zwischen 3 und 6 Gew.-% vorhanden sind.

10. Zusammensetzung gemäß einem der Ansprüche 1 bis 9 in Form von Creme, Lotion, Salbe, Spray, Lippenstift.

11. Eine bio-kompatible Sonnenschutzzusammensetzung umfassend:
- einen oder mehrere UV-Filter, ausgewählt aus 2,2'-Methylen-bis-(6-(2H-Benzotriazol-2-yl)-4-(1,1,3,3-tetramethylbutyl)-phenol, 2-[4-(Diethylamin)-2-hydroxybenzoyl]hexylbenzoat, Ethylhexyltriazon;
- ein oder mehrere Antioxidationsmittel, ausgewählt aus Tocopherol, Retinol, polyphenolischen Verbindungen, die von grünem Tee extrahiert sind;
- einen oder mehrere Träger, ausgewählt aus Propylenglykol-Dicaprylat/Dicaprat, Olivenöl, Bienenwachs;
und frei von:
- Konservierungsmitteln, die zur Paraben-Familie gehören;
- Ascorbinsäurederivaten;
- UV-Filtern, ausgewählt aus Bis-Ethylhexyloxyphenol-Methoxyphenyltriazin, Cinnamaten, Benzophenonen, Kampferderivaten, Titanoxid;
- Duftstoffen, ausgewählt aus ätherischem Melaleuca-Öl, Ylang-Ylang-Blütenextrakten, Bambousa Arundinacea-Extrakten, Karitè;
- Trägern, ausgewählt aus Fetten tierischen Ursprungs und Arganöl als Sonnenschutzmittel, das nicht schädlich für Korallenriffe ist.

## Revendications

1. Composition d'écran solaire éco-compatible, comprenant :
- les filtres UV 2,2'-méthylène-bis-(6-(2H-benzotriazole-2-yl)-4-(1,1,3,3-tétraméthylbutyl)-phénol, 2-[4-(diéthylamine)-2-hydroxybenzoyl]hexyl benzoate et éthylhexyl triazone ;
- un ou plusieurs antioxydants choisis parmi l'acétate de tocophéryle, le tocophérol, le rétinol, les composés polyphénoliques extraits du thé vert ;
- un ou plusieurs véhicules choisis parmi le dicaprylate/dicaprate de propylène glycol, l'huile d'olive, la cire d'abeille ;
et exempte de :
- conservateurs appartenant à la famille des parabènes ;
- dérivés d'acide ascorbique ;
- filtres UV choisis parmi la bis éthylhexyloxyphénol méthoxyphényl triazine, les cinnamates, les benzophénones, les dérivés de camphre, l'oxyde de titane ;
- parfums choisis parmi l'huile essentielle d'arbre à thé, les extraits de fleur d'ylang ylang, les extraits de Bambousa Arundinacea, le karité ;
- véhicules choisis parmi les graisses dérivées d'animaux et l'huile d'argan.

2. Composition d'écran solaire éco-compatible, comprenant :
- un ou plusieurs filtres UV choisis parmi le 2,2'-méthylène-bis-(6-(2H-benzotriazole-2-yl)-4-(1,1,3,3-tétraméthylbutyl)-phénol, le 2-[4-(diéthylamine)-2-hydroxybenzoyl]hexyl benzoate et l'éthylhexyl triazone ;
- un ou plusieurs antioxydants choisis parmi l'acétate de tocophéryle, le tocophérol, le rétinol, les composés polyphénoliques extraits du thé vert ;
- un ou plusieurs véhicules choisis parmi le dicaprylate/dicaprate de propylène glycol, l'huile d'olive, la cire d'abeille ;
- et comprenant en outre un ou plusieurs parfums choisis parmi l'orange, la lavande, le pamplemousse, la goyave et la noix de coco,
et exempte de :
- conservateurs appartenant à la famille des parabènes ;
- dérivés d'acide ascorbique ;
- filtres UV choisis parmi la bis éthylhexyloxyphénol méthoxyphényl triazine, les cinnamates, les benzophénones, les dérivés de camphre, l'oxyde de titane ;
- parfums choisi parmi l'huile essentielle d'arbre à thé, les extraits de fleur d'ylang ylang, les extraits de Bambousa Arundinacea, le karité ;
- véhicules choisis parmi les graisses dérivées d'animaux et l'huile d'argan.

3. Composition d'écran solaire éco-compatible, comprenant :
- un ou plusieurs filtres UV choisis parmi le 2,2'-méthylène-bis-(6-(2H-benzotriazole-2-yl)-4-(1,1,3,3-tétraméthylbutyl)-phénol, le 2-[4-(diéthylamine)-2-hydroxybenzoyl]hexyl benzoate, l'éthylhexyl triazone ;
- un ou plusieurs antioxydants choisis parmi l'acétate de tocophéryle, le tocophérol, le rétinol, les composés polyphénoliques extraits du thé vert ;
- un ou plusieurs véhicules choisis parmi le dicaprylate/dicaprate de propylène glycol, l'huile d'olive, la cire d'abeille ;
comprenant en outre de l'acide sorbique
et exempte de :
- conservateurs appartenant à la famille des parabènes ;
- dérivés d'acide ascorbique ;
- filtres UV choisis parmi la bis éthylhexyloxyphénol méthoxyphényl triazine, les cinnamates, les benzophénones, les dérivés de camphre, l'oxyde de titane ;
- parfums choisi parmi l'huile essentielle d'arbre à thé, les extraits de fleur d'ylang ylang, les extraits de Bambousa Arundinacea, le karité ;
- véhicules choisis parmi les graisses dérivées d'animaux et l'huile d'argan.

4. Composition selon la revendication 1, comprenant en outre un ou plusieurs parfums choisis parmi l'orange, la lavande, le pamplemousse, la goyave et la noix de coco et/ou l'acide sorbique.

5. Composition selon la revendication 2, comprenant en outre de l'acide sorbique.

6. Composition selon la revendication 1, se composant de :
- 2,2'-méthylène-bis-(6-(2H-benzotriazole-2-yl)-4-(1,1,3,3-tétraméthylbutyl)-phénol, 2-[4-(diéthylamine)-2-hydroxybenzoyl]hexyl benzoate et éthylhexyl triazone ;
- un ou plusieurs antioxydants choisis parmi l'acétate de tocophéryle, le tocophérol, le rétinol, les composés polyphénoliques extraits du thé vert ;
- un ou plusieurs véhicules choisis parmi le dicaprylate/dicaprate de propylène glycol, l'huile d'olive, la cire d'abeille ;
- eau.

7. Composition selon la revendication 1, se composant de :
- 2,2'-méthylène-bis-(6-(2H-benzotriazole-2-yl)-4-(1,1,3,3-tétraméthylbutyl)-phénol, 2-[4-(diéthylamine)-2-hydroxybenzoyl]hexyl benzoate et éthylhexyl triazone ;
- un ou plusieurs antioxydants choisis parmi l'acétate de tocophéryle, le tocophérol, le rétinol, les composés polyphénoliques extraits du thé vert ;
- acide sorbique ;
- un ou plusieurs véhicules choisis parmi le dicaprylate/dicaprate de propylène glycol, l'huile d'olive, la cire d'abeille ;
- eau.

8. Composition selon la revendication 1, se composant de :
- 2,2'-méthylène-bis-(6-(2H-benzotriazole-2-yl)-4-(1,1,3,3-tétraméthylbutyl)-phénol, 2-[4-(diéthylamine)-2-hydroxybenzoyl]hexyl benzoate et éthylhexyl triazone ;
- acétate de tocophéryle ;
- acide sorbique ;
- dicaprylate/dicaprate de propylène glycol, huile d'olive, et cire d'abeille ;
- eau ;
- hydroxyéthylcellulose ;
- EDTA ;
- hydroxyde de sodium.

9. Composition selon l'une quelconque des revendications 1 à 6, dans laquelle lesdits filtres UV sont présents en une concentration comprise entre 3 et 6 % en poids.

10. Composition selon l'une quelconque des revendications 1 à 9, sous la forme d'une crème, lotion, pommade, d'un spray, bâton à lèvres.

11. Composition d'écran solaire éco-compatible, comprenant :
- un ou plusieurs filtres UV choisis parmi le 2,2'-méthylène-bis-(6-(2H-benzotriazole-2-yl)-4-(1,1,3,3-tétraméthylbutyl)-phénol, le 2-[4-(diéthylamine)-2-hydroxybenzoyl]hexyl benzoate, l'éthylhexyl triazone ;
- un ou plusieurs antioxydants choisis parmi le tocophérol, le rétinol, les composés polyphénoliques extraits du thé vert ;
- un ou plusieurs véhicules choisis parmi le dicaprylate/dicaprate de propylène glycol, l'huile d'olive, la cire d'abeille ;
et exempte de :
- conservateurs appartenant à la famille des parabènes ;
- dérivés d'acide ascorbique ;
- filtres UV choisis parmi la bis éthylhexyloxyphénol méthoxyphényl triazine, les cinnamates, les benzophénones, les dérivés de camphre, l'oxyde de titane ;
- parfums choisi parmi l'huile essentielle d'arbre à thé, les extraits de fleur d'ylang ylang, les extraits de Bambousa Arundinacea, le karité ;
- véhicules choisis parmi les graisses dérivées d'animaux et l'huile d'argan en tant qu'écran solaire non nocif pour les récifs coraliens.
